(19) Europäisches Patentamt
European Patent Office
Office européen des brevets

(11) EP 2 025 680 A1

(12) **EUROPEAN PATENT APPLICATION**
published in accordance with Art. 153(4) EPC

(43) Date of publication:
**18.02.2009 Bulletin 2009/08**

(21) Application number: **06746164.0**

(22) Date of filing: **09.05.2006**

(51) Int Cl.:
***C07D 493/06*** *(2006.01)* ***A61K 31/37*** *(2006.01)*
***A61P 29/00*** *(2006.01)* ***A61P 37/08*** *(2006.01)*
***A61P 43/00*** *(2006.01)* ***C07D 493/16*** *(2006.01)*

(86) International application number:
**PCT/JP2006/309333**

(87) International publication number:
**WO 2007/129406 (15.11.2007 Gazette 2007/46)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Tropical Technology Center Ltd.**
**Uruma-shi, Okinawa 904-2234 (JP)**

(72) Inventors:
- **YASUMOTO, Takeshi**
  **Sendai-shi, Miyagi 981-0952 (JP)**
- **NAOKI, Hideo**
  **Sakai-shi, Osaka 592-8345 (JP)**
- **HIROSE, Mina**
  **Urasoe-shi, Okinawa 901-2131 (JP)**
- **ONODERA, Kenichi**
  **Okinawa-shi, Okinawa 904-2161 (JP)**
- **TSUHA, Kazuyo**
  **Okinawa-shi, Okinawa 904-2153 (JP)**
- **KUBA, Megumi**
  **Ginowan-shi, Okinawa 901-2214 (JP)**

(74) Representative: **Hartz, Nikolai**
**Wächtershäuser & Hartz**
**Patentanwälte**
**Weinstrasse 8**
**80333 München (DE)**

(54) **DEGRANULATION INHIBITOR**

(57) An object of the invention is to find in nature a compound having a degranulation inhibitory action comparable to that of steroidal or non-steroidal degranulation inhibitors, but without any side effect such as hormone action or gastrointestinal disturbances which are observed in these inhibitors, and there is provided a degranulation inhibitor comprising as an active ingredient an ellagic acid derivative of the formula (I):

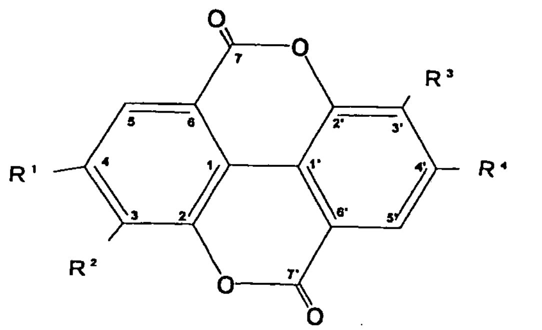

(I)

[wherein $R^1$ represents a hydroxy group or methoxy group, and $R^2$ represents a methoxy group, or $R^1$ and $R^2$ are taken together to form a methylenedioxy group, $R^3$ represents a hydroxy group or methoxy group, and $R^4$ represents a glucosyloxy group or hydroxy group]
or a salt thereof.

EP 2 025 680 A1

FIG. 1

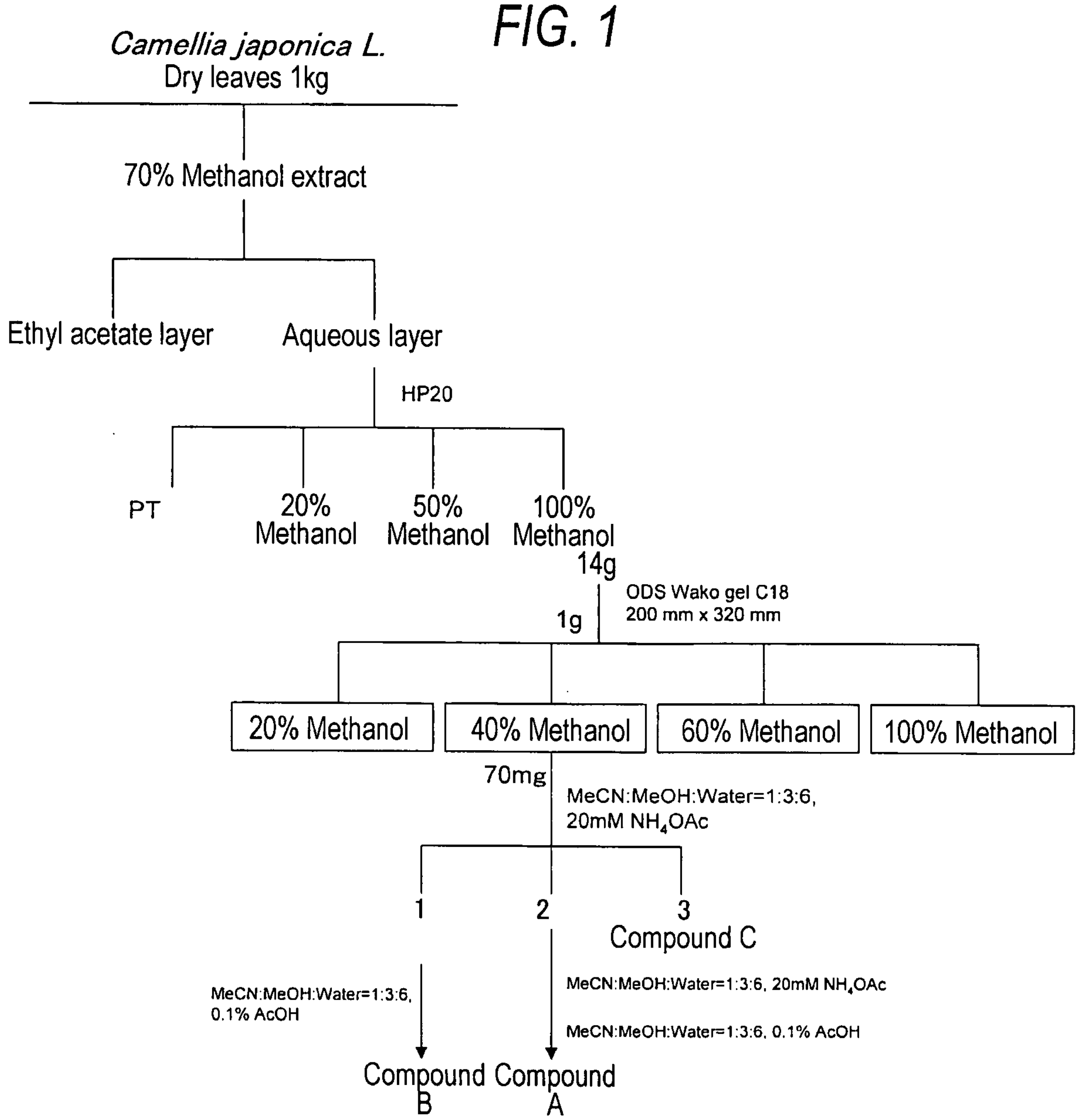
Camellia japonica L.
Dry leaves 1kg
70% Methanol extract
Ethyl acetate layer
Aqueous layer
HP20
PT
20% Methanol
50% Methanol
100% Methanol
14g
1g
ODS Wako gel C18
200 mm x 320 mm
20% Methanol
40% Methanol
60% Methanol
100% Methanol
70mg
MeCN:MeOH:Water=1:3:6,
20mM NH4OAc
1
2
3
Compound C
MeCN:MeOH:Water=1:3:6,
0.1% AcOH
MeCN:MeOH:Water=1:3:6, 20mM NH4OAc
MeCN:MeOH:Water=1:3:6, 0.1% AcOH
Compound B
Compound A

## Description

### Technical Field

The present invention relates to degranulation inhibitors. More particularly, it relates to a degranulation inhibitor containing as an active ingredient a certain ellagic acid derivative or a salt thereof.

### Background of the Invention

Drugs of steroidal and non-steroidal types have been widely used for suppression of many inflammations and allergic diseases. However, the steroidal agents have a problem of side effects such as hormone action, while the non-steroidal agents may cause clinically important enteric disorders such as gastrointestinal disorder.

In particular, drugs for allergic diseases such as pollinosis, which last for a certain period, have to be administered for a long term in many cases, and drugs with higher safety have been required, accordingly, and it has been desired to provide agents derived from natural substances which treat these diseases.

Incidentally, degranulation has been considered to be one of the reasons for inflammations and allergic diseases, and among substances inhibiting degranulation which are derived from natural products, the dimer of ellagic acid contained in the pericarp of Zakuro (*Punica granatum*) has been known and reported to be effective as an anti-inflammatory, analgesic and anti-pyretic agent (Patent document 1). In addition, a GOD type of ellagic tannin obtained from plants belonging to the family *Rosacea* has been reported to be effective as an anti-allergic agent and degranulation inhibitor (Patent document 2).

In addition to these agents, it has been disclosed that extracts from various plants such as an extract of bark of Yamamomo (*Myrica rubra*) exhibit a hexosaminidase release-inhibitory activity, which is related to inflammation (Non-Patent Document 1).

It is hard to say, however, that these so far reported ellagic acid derivatives and ellagic tannin are of practical use sufficiently, since their activity is weak. Thus, an anti-inflammatory action possessed by natural substances have continuously been studied until now in order to find out a material exhibiting a much better degranulation inhibitory action derived from natural substances.

Patent document 1: JP-A-5-310745
Patent document 2: JP-A-9-124498
Non-patent document 1: Matsuda H, Morikawa T, Tao J, Ueda K, Yoshikawa M., Chem Pharm Bull (Tokyo)., 50(2): 208-215, 2002

### Disclosure of the Invention

### Problems that the Invention is to Solve

Thus, the objective of the present invention is to find compounds from nature, which unlike the steroidal and non-steroidal degranulation inhibitors, have no side effects such as hormone action and cause no enteric disorders, and yet which exhibit a much more potent degranulation inhibitory action than steroidal or non-steroidal degranulation inhibitors, and is to provide drugs utilizing such compounds.

### Means for Solving the Problems

In order to achieve the above objective, the present inventors have intensively studied to find a compound exhibiting an excellent degranulation inhibitory action from natural substances and found that there were materials having a potent degranulation inhibitory action in the extracts of the leaves of Yabutsubaki *(Camellia japonica L.).* Further, they have worked to isolate and purify such materials, and as a result they found that the materials are certain ellagic acid derivatives. Thus, the invention has been completed.

That is, the present invention provides a degranulation inhibitor comprising as an active ingredient an ellagic acid derivative of the formula (I):

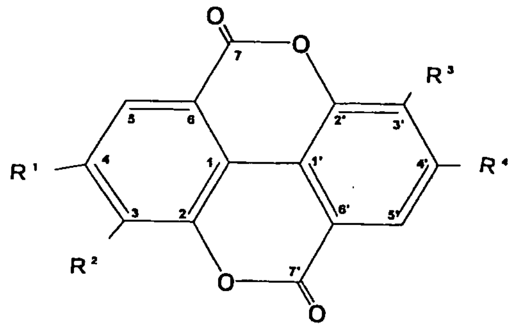

(I)

[wherein $R^1$ represents a hydroxy group or methoxy group, and $R^2$ represents a methoxy group, or $R^1$ and $R^2$ are taken together to form a methylenedioxy group, $R^3$ represents a hydroxy group or methoxy group, and $R^4$ represents a glucosyloxy group or hydroxy group]
or a salt thereof.

The invention also provides an ellagic acid glycoside of the following formula (II):

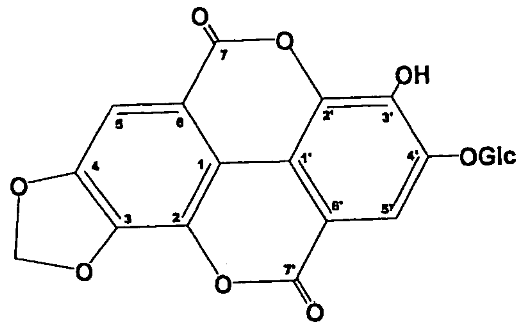

(II)

[wherein Glc represents a glucosyl group]
or a salt thereof.

Advantages of the Invention

The ellagic acid derivatives of the formula (I) in the invention have a better degranulation inhibitory action than ketotifen fumarate which is widely used. In particular, compounds represented by the formula (II), which are novel compounds, exhibit a much better degranulation inhibitory action than ketotifen fumarate.

Thus, the degranulation inhibitors comprising the formula (I) as active ingredients, especially, the degranulation inhibitors comprising as active ingredients compounds of the formula (II), can be used in treatment or prevention of a variety of inflammation and allergic diseases, for example symptoms such as pain, fever and inflammation related to influenza or other viral infections, microbe-infected pharyngitis, throat pain, bronchitis, adenoiditis, periodontitis, alveolitis, toothache, gingivitis, gout, arthritis, nephritis, hepatitis, dysmenorrhea, headache, ulcerative colitis, sprain/wrench, myalgia, neuralgia, synovitis, burn, pollinosis, bronchial asthma, atopic dermatitis, inflammation after surgical or dental treatment, and the like.

Best Modes for Carrying Out the Invention

The ellagic acid derivatives represented by the formula (II), which is one of the active ingredients of the degranulation inhibitors in the invention, are contained, for example, in the extract of *Camellia japonica L.*.

This extract of *Camellia japonica L.* can be obtained by extracting the leaves of *Camellia japonica L.* with a suitable solvent in a conventional method. The raw material *Camellia japonica L.* is a dicotyledon belonging to the family *Theaceae* and is a wild species which is also called Yamatsubaki. Most of horticultural varieties of camellia are differentiated from *Camellia japonica L.* and a lot of interspecific hybrids have been created as well as varieties. There is no particular limitation for the growing district and the collection period of the leaves of *Camellia japonica L.*. Although non-dried leaves may be used, dried leaves are usually used and the leaves are preferably ground or finely cut prior to an

extracting operation. In this connection, since the above ellagic acid derivatives (II) are contained not only in the leaves of *Camellia japonica L.* but also in those of Tsubaki *(Camellia japonica L. cv.),* Kantsubaki (*Camellia hiemalis*), etc., the extracts from *Camellia japonica L. cv.* or Kantsubaki may be utilized in place of the above extracts of *Camellia japonica* L. in order to obtain these derivatives. The growing district and the collection period of the leaves of *Camellia japonica L. cv.* or Kantsubaki which are used in extraction, similarly, is not particularly limited.

As to the solvent used for extraction of leaves of *Camellia japonica L.,* it is preferred to use water, a hydrophilic solvent or a mixture thereof. In the case of water, among them, it is preferred to use alkaline water where pH is about 8 to 12. Examples of the hydrophilic solvent include alcohols such as methanol, ethanol, propanol, isopropanol and butanol; cellosolves; ketones such as acetone; ethers such as dioxane and tetrahydrofuran; and nitrogen-containing solvents such as pyridine, morpholine, acetonitrile, N,N-dimethylformamide, N,N-dimethylacetamide and N-methylpyrrolidone. Each of those extracting solvents may be used alone, in combination of two or more, or as a mixed solvent with water.

When the hydrophilic solvent is used as a mixed solvent with water, their ratio may be appropriately selected, for example, from the range where a ratio of water/solvent is from 95/5 to 5/95(by volume; hereinafter all the mixing ratio of solvents indicated by volume ratio).

Among the above-mentioned extracting solvents, examples of the particularly preferred ones include hot water and a mixed solvent of lower alcohols such as methanol and ethanol with water, and more preferred one is a mixed liquid of a lower alcohol with water in which a lower alcohol is contained in such a ratio that water/solvent is from 30/70 to 70/30 by volume.

An extraction using the above-mentioned solvent may be carried out at appropriate temperature such as from 10°C to a refluxing temperature of the solvent or, preferably, it may be carried out at about 15 to 80°C. It is also possible to extract by means of cool percolation at room temperature. Extracting time varies depending upon extracting temperature and it is about 5 minutes to 24 hours and, preferably, from about 30 minutes to 1 hour.

In the case of the compounds (I) of the invention which are glycosides, they may be extracted as mentioned above, and the resulting extract fluid may be separated and purified by conventional methods of separation and purification.

Specific example of the methods of separating and purifying the extract fluid includes a combination of a solvent partition method with adsorption chromatography, medium pressure column chromatography, and high speed liquid chromatography, etc.

Among these methods, the solvent partition method may be carried out by adding a hydrophobic solvent to the resulting extract, followed by stirring well, wherein the hydrophobic solvent to be used includes a variety of solvents separable from water, for example, alcohols such as n-butanol, isobutanol, hexanol, octanol, 2-ethylhexanol and cyclohexanol; an aromatic hydrocarbon such as benzene, toluene and xylene; a halogenated hydrocarbon such as dichloromethane, chloroform, carbon tetrachloride, dichloroethane and trichloroethylene; ethers such as ethyl ether, isopropyl ether and butyl ether; and esters such as methyl acetate, ethyl acetate and butyl acetate. Each of those hydrophobic solvents may be used alone or in combination of two or more as a mixed solvent. Among those hydrophobic solvents, n-butanol or the like is frequently used.

Adsorption column chromatography may be carried out by passing the resulting extract or a purified product thereof through an adsorbent column of Diaion HP-20, HP-21, Sepabeads SP-825, SP-850, SP-207 (all manufactured by Mitsubishi Chemical), Sephadex LH20 (Amersham Biosciences), Amberlite XAD4, XAD16HP (Rohm & Haas), Toyopearl HW40F (Tosoh) or through a molecular sieve column, followed by separating with one or more of suitable eluents; thus, a purified extract can be obtained as a fraction having higher activity.

As to the solvent which is advantageously used in the above adsorbent column chromatography, there may be used, for example, water, a hydrophilic solvent such as methanol and ethanol or a mixed solvent thereof. In this step, two or more adsorption column chromatographies may be combined.

In addition, in medium pressure column chromatography, a method of using a column of ODS Wakogel etc. as carrier together with water or alcohol or a mixture of them as eluent in the same manner as mentioned above, may be employed. Further, in high speed liquid chromatography, a reverse phase column such as Cosmosil $5C_{18}$-AR (Nacalai Tesque), Develosil (Nomura Chemical), YMC-gel (YMC), CapsulePak (Shiseido), or TSK-GEL (Tosoh) may be used together with a mobile phase such as acetonitrile/methanol/water mixture - ammonium acetate solution, or acetonitrile/methanol/water mixture - acetic acid solution.

Among the compounds (I) of the invention, some compounds (aglycones), which are not glycosides, can readily be produced by having β-glucosidase act upon the glycoside compounds (I).

Any of the compounds (I) of the invention obtained as mentioned above have an excellent degranulation inhibitory action in comparison with commercially available ketotifen fumarate, and particularly the compounds of the formula (II), which are novel compounds, have a remarkably excellent degranulation inhibitory action.

The compounds (I) of the invention, accordingly, can be used as degranulation inhibitors in, for example, anti-inflammatory agents, anti-allergic agents, etc., in combination with other known pharmaceutical carriers.

The degranulation inhibitors can be formulated into oral preparations such as tablets, capsules, powders, granules, liquids or syrups, or parenteral preparations for injection or infusion, or inhalations, aerosols, external preparations, plasters, or the like.

The pharmaceutical carriers which can be used in production of the above-mentioned respective preparations are exemplified by: widely known solid carriers including excipients such as starch, lactose, sucrose, mannitol, corn starch, crystalline cellulose, carboxymethyl-cellulose, sugar silicate; binders such as polyvinyl alcohol, polyvinylpyrrolidone, polyvinyl ether, ethylcellulose, gum arabic, tragacanth, gelatin, hydroxypropylcellulose, dextrin, or pectin; lubricants such as magnesium stearate, talc, or polyethylene glycol; disintegrators; disintegration coagents; and stabilizers; or carriers for liquid preparations including liquid ingredients such as water, ethyl alcohol, ethylene glycol, or glycerin; surfactants such as polyoxyethylene sorbitan fatty acid ester; taste components such as glucose or amino acids; solubilizing agents; coloring agents; and preservatives. For inhalations, aerosols, external preparations, and plasters, known carriers suited to these formulations may be employed.

The amount of the compounds (I) to be blended into the granulation inhibitors of the invention depends on the kind, intended use and symptoms, but the daily dose for an adult is preferably in the range of about 0.01 μg to 10 mg, in particular, preferably about 0.1 μg to 1 mg.

Further, the compounds (I) of the invention may be used as food additives which may be added to common food/beverage or healthy foods in combination with other food materials.

Examples

The present invention will be explained in more detail by the following Examples and test examples although the present invention is not limited by those Examples, etc. at all.

Example 1

Preparation of ellagic acid derivatives from the extract of *Camellia japonica L.*

Leaves (2 kg) of *Camellia japonica L.* (produced in Okinawa) were dried at 60°C for 2 - 3 hours, and then ground to about 3 - 6 mm in width with a mixer. To the ground product (about 1 kg) of the leaves of *Camellia japonica L.* was added about 10 L (about ten times by weight of the leaves) of a mixed liquid of water/methanol (3/7), and the mixture was stirred with a homogenizer for grinding and extraction for 2 minutes. The extract was centrifuged at 3,000 rpm for 10 minutes at 4°C and the supernatant thus obtained was collected. To the residue was added 5 parts by weight of water/methanol (3/7), and 3 parts by weight in order; and the same operation was repeated twice. The resulting supernatants were combined, filtered through a filter paper, and concentrated under reduced pressure to obtain about 6 L of filtrate.

Ethyl acetate (1 L) was added to 2 L of the solution concentrated under reduced pressure and the solution was distributed by shaking at room temperature; this operation was repeated twice. After all of the filtrates were distributed, the resulting aqueous layer and ethyl acetate layer were respectively concentrated under reduced pressure. The aqueous layer was further concentrated under reduced pressure to obtain about 5.6 L of the filtrate.

The resulting filtrate (aqueous layer) was adsorbed on a resin adsorption column (Ø90 mm x 170 mm) using HP20 (Diaion, about 1 L) as carrier, and successively eluted with 3 L of water/methanol (8/2; by volume, hereinafter same), 3 L of water/methanol (5/5) and 3 L of methanol, and each fraction was evaporated to solidity under reduced pressure.

Solid material (14 g) was obtained from the methanol-eluted fraction (methanol fraction), and 1 g of the resultant was dissolved in 15 mL of water, applied to medium pressure column chromatography (ODS Wakogel C18, 20 mm x 320 mm), and successively eluted with 600 mL of water/methanol (8/2), 600 mL of water/methanol (6/4), 600 mL of water/methanol (4/6) and 600 mL of methanol (flow rate = 12 mL/min).

Thus resulting water/methanol (6/4) fraction was applied to high speed liquid chromatography (column: Cosmosil $5C_{18}$-AR; 10 mm in diameter, 250 mm in length), eluted with acetonitrile/methanol/water (1/3/6) - 20mM ammonium acetate as mobile phase (flow rate = 2.5 mL/min), and detected using UV at 365 nm and 280 nm to obtain fractions 1, 2 and 3. Among these, from the fraction 3, 4 mg of 3,3',4-tri-O-methylellagic acid (Compound (C)) was obtained. The fraction 2 was further fractionated under the above-mentioned conditions and purified with acetonitrile/ methanol/water (1/3/6) - 0.1% acetic acid as mobile phase to obtain 0.2 mg of a novel compound, 3,4-dioxoloellagic acid 4'-glucoside (Compound (A)). The fraction 1 was purified with acetonitrile/ methanol/water (1/3/6) - 0.1% acetic acid as mobile phase to obtain 0.4 mg of 3-O-methylellagic acid 4'-glucoside (Compound (B)). Additionally, 2 mg of Compound (A) and Compound (B) were obtained respectively by repeating the above operation. The above process is summarized in Fig. 1.

Structure and physicochemical properties of Compound (A):

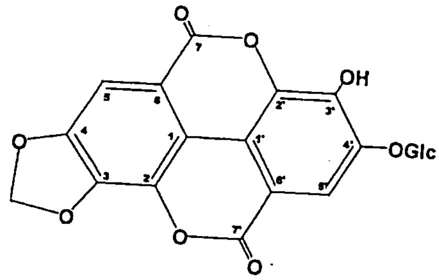

(A)

(1) Color of material: pale yellow
(2) Molecular weight: 476
(3) Molecular formula: $C_{21}H_{16}O_{13}$
(4) Mass spectrum: HRMS (MALDI-TOF negative)
Found, m/z 475.0517 $[M-H]^-$
Calcd for $C_{21}H_{15}O_{13}$ 475.0507
(5) Optical rotation: $[\alpha]_D^{24}$ -92° (c 0.05, $H_2O$)
(6) $^1$H-NMR (measured in heavy water; 600MHz) δ ppm:
7.06 (1H, s), 6.73 (1H, s), 5.99 (2H, d, J=13Hz), 4.82 (1H, d, J=7Hz), 3.90 (1H, brd, J=12Hz), 3.73 (1H, dd, J=12,5Hz), 3.62-3.52 (3H, m), 3.46 (1H, t, J=9Hz)
(7) $^{13}$C-NMR (measured in heavy water; 150MHz) δ ppm:
161.5, 160.6, 153.1, 151.0, 150.9, 138.9, 137.3, 131.0, 115.9, 114.3, 112.6, 111.5, 105.7, 104.6, 102.3, 98.8, 77.0, 76.3, 73.9, 70.5, 61.6

Structure and physicochemical properties of Compound (B):

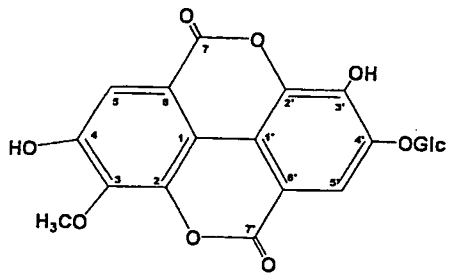

(B)

(1) Color of material: pale yellow
(2) Molecular weight: 478
(3) Molecular formula: $C_{21}H_{18}O_{13}$
(4) Mass spectrum: EIMS negative
Found, m/z 477$[M-H]^-$
(5) $^1$H-NMR (measured in heavy water, 600MHz) δ ppm:
7.11 (1H, s), 6.89 (1H, s), 4.88 (1H, d, J=7Hz), 3.91 (1H, dd, J=13,2Hz), 3.87(3H, s), 3.71 (1H, dd, J=13,5Hz), 3.62-3.55 (3H, m), 3.44 (1H, t, J=9Hz)
(6) $^{13}$C-NMR (measured in heavy water, 150MHz) δ ppm:
165.2, 164.9, 157.6, 153.8, 149.3, 143.1, 142.9, 140.3, 118.3, 115.8, 115.1, 114.6, 114.5, 104.9, 100.8, 79.8, 79.0, 76.7, 73.3, 64.9, 64.4

Structure and physicochemical properties of Compound (C):

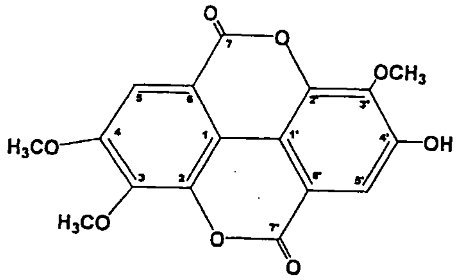

(C)

(1) Color of material: pale yellow
(2) Molecular weight: 344
(3) Molecular formula: $C_{17}H_{12}O_8$
(4) Mass spectrum: EIMS negative
Found, m/z 343$[M-H]^-$
(5) $^1$H-NMR (measured in dimethylsulfoxide, 600MHz) δ ppm:
8.23 (1H, s), 7.67 (1H, s), 4.12 (3H, s), 4.05 (3H, s), 4.02 (3H, s)
(6) $^{13}$C-NMR (measured in dimethylsulfoxide, 150MHz) δ ppm:
158.5, 158.3, 154.4, 147.6, 143.3, 141.4, 140.9, 140.9, 117.6, 114.1, 112.9, 112.8, 111.5, 107.5, 61.5, 61.3, 56.7

Example 2

Preparation of an aglycone from an ellagic acid derivative:

First, 1 mL of 50mM phosphate buffer (pH 6.0) was added to 300 μg of Compound (A). On the other hand, the phosphate buffer was added to β-glucosidase (Oriental Yeast) so that concentration was 1 mg/mL. 200 μL each of the solution of Compound (A) and the solution of β-glucosidase, were mixed, and the mixture was incubated at 37°C for 1 hour. After the reaction completion, the mixture was centrifuged at 13,000G for 10 minutes at 4°C. The supernatant was filtered through a filter (by Wattman; PVDF, pore size 0.45 μm), and the state of reaction was confirmed by means of LC/MS, indicating that the product was an aglycone of Compound (A), i.e. 3,4-dioxoloellagic acid represented by the following formula (D).

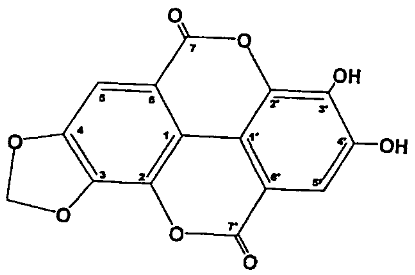

(D)

Example 3

Measurement of degranulation inhibitory activity:

With regard to the measurement of degranulation inhibitory activity, a test for hexosaminidase release-inhibitory activity was carried out by referring to Non-Patent Document 1 and Non-Patent Document 2 (Kataoka M., Takagaki Y., Shoyakugaku Zasshi, 46(1), 25-29, 1992). Firstly, rat basophilic leukemia cells (RBL-2H3) were made into 5 x $10^5$ cells/mL and seeded on a 96-well plate and anti-DNP-BSA mouse IgE antibody was added thereto so as to make its final concentration 0.29 μg/mL and incubated with 5% $CO_2$ at 37°C overnight in an incubator to sensitize the cells. Then the cells were washed with a phosphate-buffered physiological saline solution twice and 130 μL of a releasing mixture

(comprising 116.9 mM of NaCl, 5.4 mM of KCl, 0.8 mM of $MgSO_4$, 2.0 mM of $CaCl_2$, 5.6 mM of glucose, 0.1% of bovine serum albumin and 25 mM of HEPES) was added thereto.

Then, Compounds (A) and (C) obtained in the invention were first dissolved in 25% ethanol, and then Compound (A) was diluted with 1% ethanol to achieve the final concentration in 7 serial dilutions in steps of from 1 μg/mL to 5 ng/mL; and Compound (C) was diluted with 1% ethanol to achieve the final concentration by 7 serial dilutions in steps of from 10 μg/mL to 50 ng/mL. Compound (B) was first dissolved in water, and then diluted with 1% ethanol to achieve the final concentration by 7 serial dilutions in steps of from 50 μg/mL to 50 ng/mL. Compound (D) was diluted with 4% ethanol-20mM phosphate buffer to achieve the final concentration by 4 serial dilutions in steps of 2.6 μg/mL to 165 ng/mL. 10 μL portion samples of all concentrations of Compounds (A), (B), (C) and (D) were added and allowed to stand at 37°C under 5%$CO_2$ in an incubator for 10 minutes. Then 10 μL of an antigen DNP-BSA (2 μg/mL) was added, the mixture was allowed to stand in an incubator for 1 hour to induce degranulation, and centrifuged to collect the supernatant. 15 μL of a 5mM hexosaminidase substrate solution (p-nitrophenyl-β-D-glucosaminide) was added to 45 μL of the supernatant liquid, the mixture was made to react at 37°C for 3 hours and 180 μL of a solution for stopping the reaction (0.1M $NaHCO_3/Na_2CO_3$; pH 10.0) was added thereto. After completion of the reaction, absorbance at 415 nm was measured and the hexosaminidase release-inhibitory activity was calculated by the following formula. The results for Compounds (A), (B), (C) and (D) are shown in Table 1. Meanwhile, a positive control (200 μM of ketotifen fumarate) and a negative control corresponding to the final solvent concentration for the test substance were prepared.

$$\text{Hexosaminidase release-inhibitory Activity(\%)} = [1 - (S - B/C - b)] \times 100$$

S: absorbance of the test substance upon addition of cells
B: absorbance upon addition of the test substance in the absence of the cells
C: absorbance of the negative control
b: absorbance in the absence of cells

[Table 1]

| Sample | | $IC_{50}$ value |
|---|---|---|
| Product of the Invention | Compound (A) | 6.63 ng/mL (14 nM) |
| | Compound (B) | 14.83 μg/mL (31 μM) |
| | Compound (C) | 4.47 μg/mL (13 μM) |
| | Compound (D) | 1.07 μg/mL (3 μM) |
| Ketotifen fumarate | | 71.75 μg/mL (169 μM) |

As described in Table 1, the inhibition of release of hexosaminidase ($IC_{50}$ value) was 6.63 ng/mL for Compound (A), 14.83 μg/mL for Compound (B), 4.47 μg/mL for Compound (C), and 1.07 μg/mL for Compound (D).

From these results, it was found that the $IC_{50}$ value of Compound (A) is at least 10,000 times higher than the positive control ketotifen fumarate, and Compounds (B), (C) and (D) all also have a higher activity.

Industrial Applicability

In inflammation and its causative allergic reaction, there are generally four types such as anaphylaxis (type I), cytotoxic type (type II), Arthus type (type III) and cell-mediated type (delayed type) (type IV). Pollinosis which has been particularly becoming a problem in recent years is classified under the type I allergy (immediate type allergy).
Although it has been said that atopic dermatitis mainly comprises the type I allergic reaction as well, it has been found recently that the type IV allergic reaction also participates in that.

Reaction mechanism of this type I (immediate type) allergy is that IgE produced by B cells is bonded to a highly affinitive IgE receptor existing on cell membrane of mast cells -basophiles, and exogenous antigen cross-links to IgE on cell membrane whereupon a mediator such as histamine or leukotriene is released to result in onset of allergy. Since

hexosaminidase is released as a granulation material together with histamine, hexosaminidase may be used as an indicator of histamine release. Therefore, in order to prevent the type I allergic reaction, any of the above pathways is to be cut.

This being the case, since Compounds (I) of the invention have an excellent degranulation inhibitory activity as shown in the above examples, Compounds (I) are very effective in treatment or prevention of diseases caused by inflammation.

Therefore, the degranulation inhibitors comprising the above-mentioned Compounds (I) as active ingredients can be used as drugs for human and animals or as additives to a variety of food/beverage including healthy foods.

Brief Description of Drawings

Fig. 1 shows a process for producing Compounds of the invention.

## Claims

1. A degranulation inhibitor, comprising, as an active ingredient, an ellagic acid derivative of the formula (I):

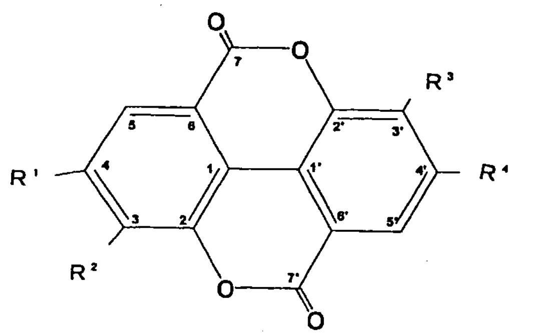

(I)

[wherein $R^1$ represents a hydroxy group or methoxy group, and $R^2$ represents a methoxy group, or $R^1$ and $R^2$ are taken together to form a methylenedioxy group, $R^3$ represents a hydroxy group or methoxy group, and $R^4$ represents a glucosyloxy group or hydroxy group] or a salt thereof.

2. A degranulation inhibitor according to Claim 1, comprising, as an active ingredient, a compound of the formula (I), wherein the groups $R^1$ and $R^2$ are taken together to form a methylenedioxy group.

3. A degranulation inhibitor according to Claim 1 or 2, being an anti-inflammatory agent.

4. A degranulation inhibitor according to Claim 1 or 2, being an anti-allergic agent.

5. A degranulation inhibitor according to any one of Claims 1 to 4, being an orally administrable preparation.

6. An ellagic acid glycoside of the following formula (II) :

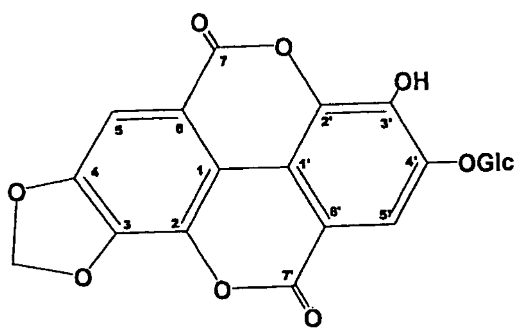

(II)

[wherein Glc represents a glucosyl group]
or a salt thereof.

FIG. 1

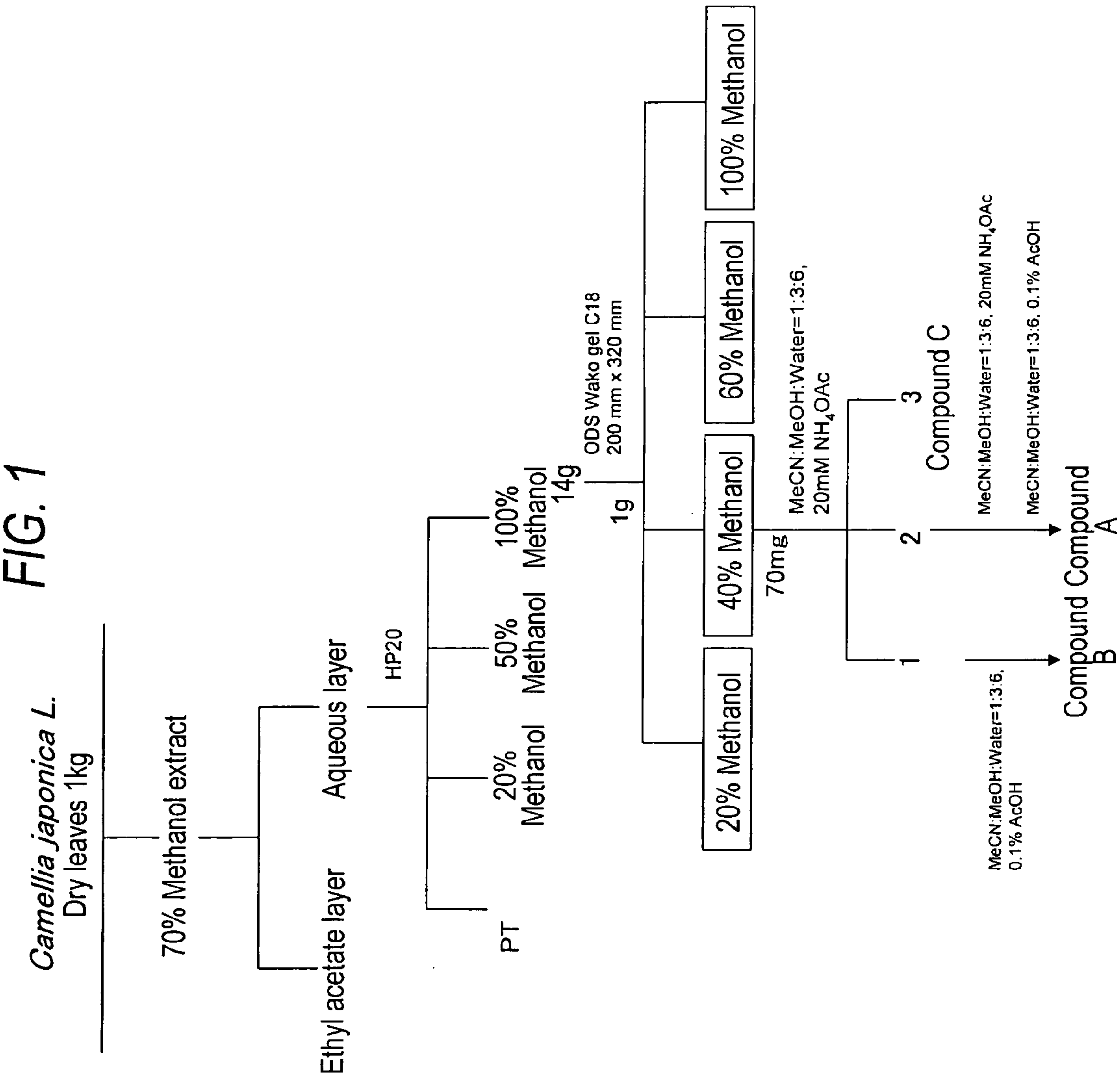
Camellia japonica L.
Dry leaves 1kg
70% Methanol extract
Ethyl acetate layer
Aqueous layer
HP20
PT
20% Methanol
50% Methanol
100% Methanol
14g
ODS Wako gel C18
200 mm x 320 mm
1g
20% Methanol
40% Methanol
60% Methanol
100% Methanol
70mg
MeCN:MeOH:Water=1:3:6,
20mM NH4OAc
1
2
3
Compound C
MeCN:MeOH:Water=1:3:6,
0.1% AcOH
MeCN:MeOH:Water=1:3:6, 20mM NH4OAc
MeCN:MeOH:Water=1:3:6, 0.1% AcOH
Compound B
Compound A

**INTERNATIONAL SEARCH REPORT**

International application No. PCT/JP2006/309333

A. CLASSIFICATION OF SUBJECT MATTER
*C07D493/06*(2006.01)i, *A61K31/37*(2006.01)i, *A61P29/00*(2006.01)i, *A61P37/08*(2006.01)i, *A61P43/00*(2006.01)i, *C07D493/16*(2006.01)i

According to International Patent Classification (IPC) or to both national classification and IPC

B. FIELDS SEARCHED

Minimum documentation searched (classification system followed by classification symbols)
C07D493/06, A61K31/37, A61P29/00, A61P37/08, A61P43/00, C07D493/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 02-231408 A (Lion Corp.),<br>13 September, 1990 (13.09.90),<br>Full text; particularly, example 4<br>(Family: none) | 1-5<br>6 |
| X<br>Y | JP 02-231423 A (Lion Corp.),<br>13 September, 1990 (13.09.90),<br>Full text; particularly, example 4<br>(Family: none) | 1-5<br>6 |
| X<br>Y | JP 2004-359732 A (Kabushiki Kaisha Ryukyu Bio Resource Kaihatsu),<br>24 December, 2004 (24.12.04),<br>Full text; particularly, tables 1, 2<br>(Family: none) | 1-5<br>6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>08 August, 2006 (08.08.06) | Date of mailing of the international search report<br>15 August, 2006 (15.08.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/309333

C (Continuation). DOCUMENTS CONSIDERED TO BE RELEVANT

| Category* | Citation of document, with indication, where appropriate, of the relevant passages | Relevant to claim No. |
|---|---|---|
| X | ZHOU, Guangwei et al., Studies on chemical constituents from Semiliquidambar cathayensis, Zhongcaoyao, 2002, 33(7), pages 589 to 591 | 1-5 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5310745 A
- JP 9124498 A


### Non-patent literature cited in the description


- **MATSUDA H ; MORIKAWA T ; TAO J ; UEDA K ; YOSHIKAWA M.** *Chem Pharm Bull (Tokyo).,* 2002, vol. 50 (2), 208-215
- **KATAOKA M. ; TAKAGAKI Y.** *Shoyakugaku Zasshi,* 1992, vol. 46 (1), 25-29